Europäisches Patentamt

European Patent Office

Office européen des brevets

(11) Publication number: **0 461 477 A1**

# EUROPEAN PATENT APPLICATION

(21) Application number: 91108811.0

(51) Int. Cl.⁵: **C12Q 1/68**

(22) Date of filing: 29.05.91

(30) Priority: 31.05.90 JP 144195/90
31.05.90 JP 144196/90

(43) Date of publication of application:
**18.12.91 Bulletin 91/51**

(84) Designated Contracting States:
**CH DE FR GB IT LI**

(71) Applicant: **SHIMADZU CORPORATION**
**1, Nishinokyo-Kuwabaracho**
**Nakagyo-ku Kyoto-shi Kyoto 604(JP)**

(72) Inventor: **Yamagata, Koichi**
**11-25-105, 1-chome, Komatsu,**
**Higashiyodogawa-ku**
**Osaka-shi, Osaka-fu(JP)**
Inventor: **Shirasaki, Yoshinari**
**3-25-13, Shigasato**

Otsu-shi, Shiga-ken(JP)
Inventor: **Ohashi, Tetsuo**
**18, Shunei-cho, Saiin, Ukyo-ku**
**Kyoto-shi, Kyoto-fu(JP)**
Inventor: **Tada, Jun**
**16, Nanjo, Mozume-cho**
**Mukou-shi, Kyoto-fu(JP)**
Inventor: **Fukushima, Shigeru**
**4-25-5, Ichiriyama**
**Otsu-shi, Shiga-ken(JP)**
Inventor: **Kita, Junichi**
**3-7-4, Kitakutsukake-cho, Ohe, Nishigyo-ku**
**Kyoto-shi, Kyoto-fu(JP)**

(74) Representative: **Vossius & Partner**
**Siebertstrasse 4 P.O. Box 86 07 67**
**W-8000 München 86(DE)**

(54) **Method of bacterial examination and apparatus therefor.**

(57) The present invention is directed to a method of bacterial examination to determine whether or not a target bacterium is present in a sample (1) which comprises collecting a bacterial cell component from a suspension containing bacteria in a filter (4), amplifying a DNA contained in said bacterial cell component by polymerase chain reaction and detecting said amplified DNA, and an apparatus for performing the above method.

The method of the present invention makes it possible to quickly, conveniently, safely and cheaply obtain the DNA-containing bacterial cell component from urine samples and even from fecal samples and offers convenience for the method of bacterial examination, thus permitting identification of the target pathogenic bacterium in a short time.

FIG. 1

Rank Xerox (UK) Business Services

The present invention relates to a method of bacterial examination and an apparatus therefor. More specifically, the present invention relates to a method of bacterial examination comprising collecting pathogenic bacteria from various samples such as urine samples containing bacteria and fecal suspension samples containing food poisoning bacteria and extracting and examining the DNA from the bacterial cell component, and an apparatus therefor.

In the clinical diagnosis and research fields, it has long been a common practice to cultivate bacteria on agar plates by various methods to identify the bacterial species in biological samples or to analyze their components.

In the conventional methods of obtaining the bacterial cell component by lysing the bacteria in urine samples or by lysing the food poisoning bacteria in fecal samples and other biological samples containing an insoluble solid such as food residues and enteric abrasive cells, a sample suspension is cultivated in medium for several days, and the resulting colonies are collected and centrifuged to separate and recover bacterial cells.

The recovered cells are lysed with an enzyme such as proteinase K, a surfactant such as sodium dodecyl sulfate or an alkali such as sodium hydroxide, or disrupted by a physical disruption means such as ultrasonication or mill to yield the bacterial cell component.

Examples of the bacterial colony isolation identification methods include the bacterial colony isolation method, in which the sample described above is sown on agar plates or another medium containing a particular antibiotic and the resulting colonies are separated and again sown on a medium containing another antibiotic and this separation is repeated in cycles, the latex agglutination method, in which an antibody specific to the target bacterium is immobilized on latex beads and added to the sample described above and identification is made on the basis of bead agglutination due to bacterial cell recognition by the antibody on the bead surface, the sandwich antibody method, in which a primary antibody is bound to polyethylene plastic beads, the resulting conjugate is reacted to bacterial cells to separate the bacterial cells which bind with the primary antibody by antigen-antibody reaction and those which do not bind therewith, and a secondary antibody labeled with enzyme or fluorescent substance is reacted and the enzyme activity or fluorescence intensity is determined after B/F separation, and the DNA probe method, in which the colony described above is transferred to a membrane filter and lysed, after which a DNA probe prepared by labeling a specific DNA fragment with enzyme or radioactive substance is reacted and the target bacterium is detected at the gene level. The DNA probe method detects the target bacterium on the basis of the formation of hydrogen bond between the DNA probe and the base sequence complementary thereto in the DNA of the colony.

However, the above-mentioned methods of obtaining the bacterial cell component are all faulty in that they do not permit quick obtainment of the bacterial cell component because much time is required for cultivation. Particularly, in the case of, for example, bacteria which cause urinary tract infection or food poisoning, early identification followed by administration of an antibiotic etc. has significant preventive and therapeutic effects on the pathogenic bacterium. Also, the nucleic acid purification method, which has traditionally been commonly used for the purpose of research, requires costly apparatuses such as a centrifuge and dangerous organic solvents such as phenol and chloroform, and is thus difficult to use in actual clinical situations.

It is an object of the present invention to provide a method of bacterial examination which is capable of conveniently, safely and cheaply identifying pathogenic bacteria and other various bacteria.

It is another object of the present invention to provide an apparatus for performing the method of bacterial examination of the present invention.

Accordingly, the method of bacterial examination of the present invention comprises collecting the bacterial cell component from a suspension containing bacteria in a filter, subjecting a solution containing the bacterial cell component described above to polymerase chain reaction to amplify the DNA of the target bacterium and using the amplified DNA to examine the target bacterium in the suspension described above.

The bacterial examination apparatus for performing the method of bacterial examination of the present invention comprises means for collecting the bacterial cell component from a suspension containing bacteria in a filter, means for amplifying the DNA in a solution containing the collected bacterial cell component and means for detecting the amplified DNA. More specifically, the apparatus of the present invention comprises means for aspirating a sample solution into a syringe equipped with a filter, means for conveying said syringe on the treating line to collect a bacterial cell component from said sample solution, means for amplifing a DNA contained in said bacterial cell component by polymerase chain reaction and means for detecting said amplified DNA.

Figure 1 illustrates the course of the method of bacterial separation and bacterial cell component collection carried out in Example 1.

Figure 2 shows the electrophoretic pattern obtained in Example 1.

Figure 3 shows the electrophoretic pattern obtained in Example 2.

Figure 4 shows an example mode of the bacterial examination apparatus based on the detection method of the present invention.

Figure 5 schematically illustrates the pretreatment portion of the bacterial examination apparatus shown in Figure 4.

The reference numbers denote the following;

1: sample solution, 2 and 3: syringe, 4: filter, 5: lytic solution, 6: cap, 7: water bath, 10: a group of vessels for pretreatment, 11: vessel containing sample suspension, 12: washing solution, 13: vessel containing lytic solution, 14: vessel containing ethanol, 15: filter, 16: syringe, 17: piston, 18: constant temperature chamber, 19: vessel for elution, 20: vessel for PCR process, 21: incubator, 22: capillary zone electrophoresis(CZE)

In the method of bacterial examination of the present invention, the bacterial cell component is first collected from a suspension containing bacteria in a filter. This process is carried out by passing the suspension through a filter holder, separating the bacterium from the suspension by means of the filter set in the filter holder, and then a lytic solution is passed through the filter to separate the bacterial cell component in the case of samples with a low foreign matter content such as urine samples in, for example, detection of bacteria which cause urinary tract infection.

The use of polymerase chain reaction for the obtained bacterial cell component by the standard method offers quickness and convenience for the method of bacterial examination. In addition, quick obtainment of results makes it possible to administer an antibiotic which specifically acts on the target pathogenic bacterium.

In the case of fecal and other samples containing an insoluble solid such as food residue or enteric abrasive cells for detection of, for example, food poisoning bacteria from feces etc., the method of bacterial examination of the present invention requires a pretreatment such as separation of these insoluble solids.

For the pretreatment, the suspension containing bacteria is first passed through the first coarse filter to separate the insoluble solids. Next, unlike the case of urine and other samples described above, a lytic agent is added to the filtrate to liberate the bacterial cell component, followed by removal of the finer particles of the insoluble solids through the second filter. To the filtrate thus obtained a nucleic acid component agglutinin is added to precipitate the nucleic acid component, followed by filtering through the third filter to capture the nucleic acid component, i.e., the bacterial cell component in the third filter. After the bacterial cell component is collected in the filter in the same manner as above, the filter is washed with a 70% aqueous solution of ethanol and then further washed with a 99% aqueous solution of ethanol, after which it is irrigated with water or an aqueous solution of surfactant to elute the nucleic acid component.

The use of polymerase chain reaction for the eluted nucleic acid component by the standard method in the same manner as above offers quickness and convenience for the method of bacterial examination of the present invention.

The primer used for PCR is an oligonucleotide which is capable of hybridizing to the DNA of the target pathogenic bacterium. The detection method for the amplified DNA is not subject to limitation with respect to DNA detection means; for example, it can be detected by fluorescence intensity such as ethidium bromide staining, chemiluminescence, or enzyme activity after agarose gel or capillary zone electrophoretic separation.

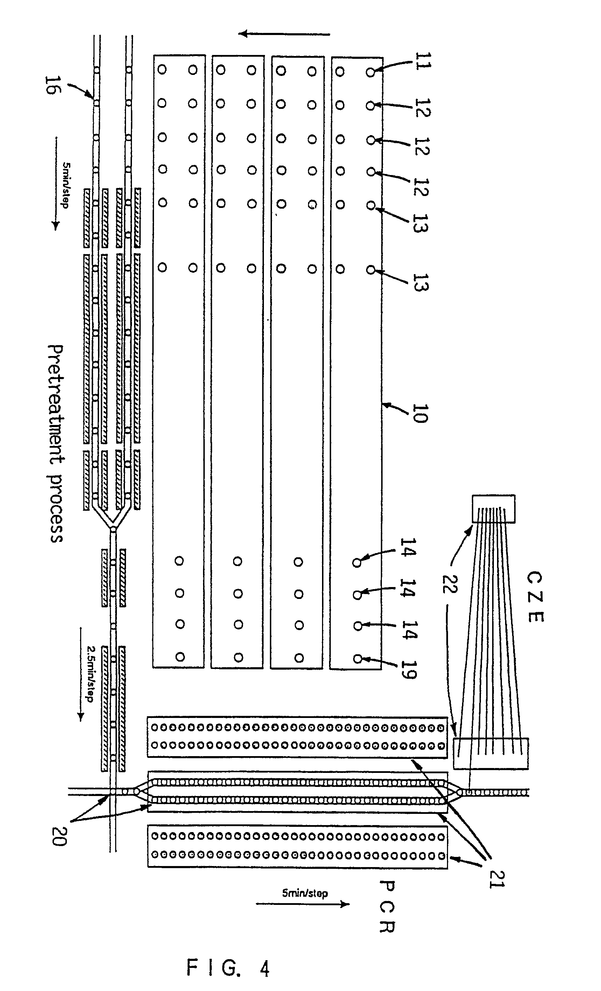

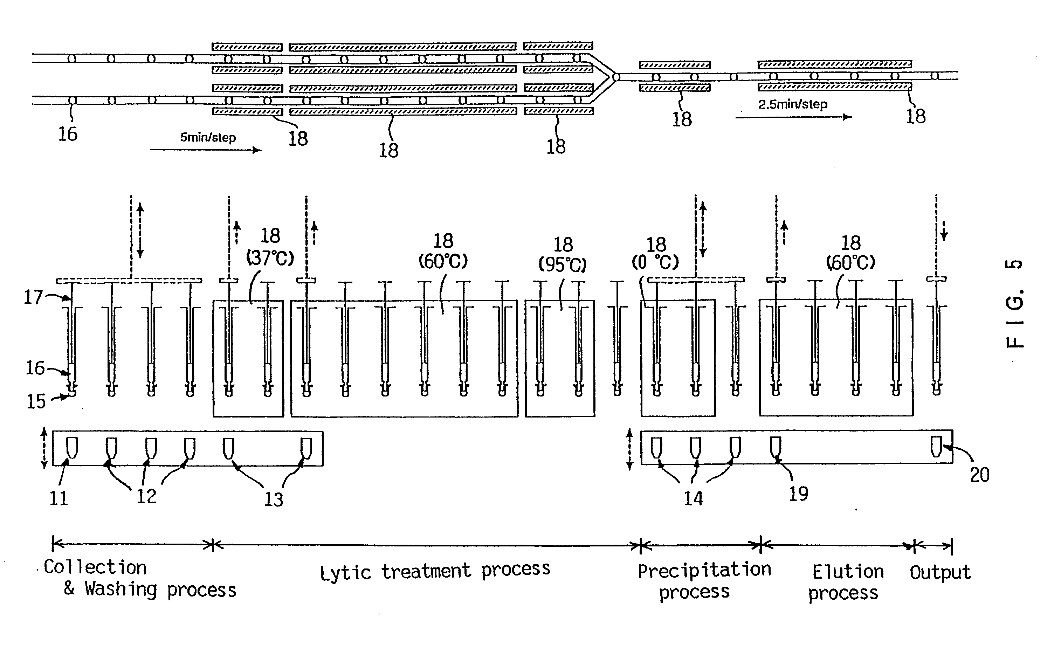

An example mode of the apparatus which automatically performs the method of bacterial examination of the present invention is schematically illustrated in Figures 4 and 5. It has a syringe 16 equipped with a filter 15 in the bottom opening thereof. A group of vessels 10 for pretreatment arranged below the syringe is upward transferred until the syringe tip enter the vessel, and the piston 17 in the syringe is enabled to aspirate the sample solution 11 in the vessel into the syringe, and subsequently the group of vessels 10 for pretreatment is downward returned to the starting position.

The filter 15 in this apparatus is composed of two kinds of glass fiber filter.

After this state is maintained for a given time (e.g., 5 minutes for one step), the syringe is conveyed to the adjoining position on the treating line and the sample suspension 11 aspirated in the previous step is discharged by the upward transfer of the group of vessels 10 and by the action of the piston 17 in the syringe 16, after which the bacteria collected in the filter is washed by aspiration of the washing solution 12.

The group of vessels 10 comprises a number of vessels containing respective treating solutions necessary to collect the bacterial cell component and obtain nucleic acid, such as a vessel containing the sample suspension 11, a vessel containing the washing solution 12, a vessel containing the lytic solution 13, a vessel containing ethanol 14 for ethanol precipitation and a vessel for elution 19, which vessels are arranged in the order of treatment.

This series of steps makes it possible to automate the aspiration of sample solution in the syringe,

3

washing of the bacteria, lytic treatment of the bacteria collected in the filter, elution of the nucleic acid component and other operations.

The syringe is passed through a constant temperature chamber 18 so that it is kept at a temperature suitable for each treatment, for example, 37 °C , 60 °C or 95 °C in the lytic treatment process, 0 °C in the ethanol precipitation process and 60 °C in the DNA elution process. Such syringe conveyance is achieved by, for example, a belt conveyor.

The DNA thus obtained is discharged in a vessel 20 for PCR process and several agents necessary for polymerase chain reaction such as two oligonucleotide primers that hybridize to opposite strands of the DNA, Taq polymerase and deoxynucleotide triphosphates (dATP, dCTP and dTTP) are added together with said DNA therein.

The vessel 20 for PCR process is passed through a incubator 21 so that it is controlled at a temperature suitable for each reaction of PCR. Specifically, in the PCR process of the present invention, three incubators are arranged; for example, one is controlled at a temperature of 94 °C for the thermally denaturing reaction, another is 37 °C for the dealing reaction and the other is 55 °C for the primers extending reaction. Such vessel conveyance through incubators is intermittently achieved by means of, for example, a belt conveyor. More concretely, a vessel is maintained for a given time (e.g., 5 minutes for one step) followed by conveyance to the adjoining position on the PCR line by the above means.

Here, the incubator 21 is arranged below a group of vessels on the PCR line, wherein each vessel composed of the group of vessels means the above mentioned vessel 20 for PCR process intermittently conveyed by means of the belt conveyor and the like. Each incubator is exchanged by turns so that PCR can be performed in the order of thermally denaturing reaction, the annealing reaction and the primers extending reaction. Such conveyance of a incubator is achieved by, for example, a cart.

The DNA thus amplified is then subjected to the detection process by an electrophoretic apparatus such as capillary zone electrophoresis (CZE) 22.

The method of the present invention makes it possible to quickly, conveniently, safely and cheaply obtain the DNA-containing bacterial cell component from urine samples and even from biological samples with a very high impurity content such as fecal samples and offers convenience for the method of bacterial examination because it is based on polymerase chain reaction, thus permitting identification of the target pathogenic bacterium in a short time within 5 hours.

EXAMPLES

The present invention is hereinafter described in more detail by means of the following examples, but the invention is not to be interpreted as limited by these examples.

Example 1

(1) Bacterial separation process

Preparation of sample suspension

$10^5$ cells of the target bacterium were suspended in 1 ml of human urine to yield a sample suspension. The target bacterium was Escherichia coli ATCC 23519 or Proteus vulgaris JCM 1668.

Filtration

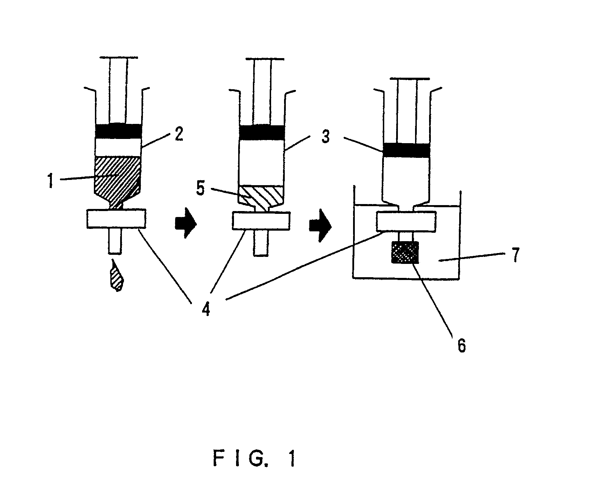

The sample suspension thus obtained was subjected to filtration. First, the sample suspension 1 was placed in the syringe 2 as shown in Figure 1, and filtered through the filter 4 connected to the syringe 2. Then, the filter was washed with 5 ml of a 50 mM phosphate buffer, pH 7.0, (hereinafter referred to as PB).

(2) Bacterial cell component collection process

Lytic treatment

Next, 250μ l of the lytic solution 5 (a solution of 1 mg/ml proteinase K, 0.13% SDS and 0.13% SLS in PB) was placed in the syringe 3, and the syringe was connected to the filter 4 (MILLEX-PF 0.8 μ m filter unit, produced by Millipore). The lytic solution 5 was introduced into the filter 4 by the action of the syringe 3. After incubation in the water bath 7 at 60 °C for 10 minutes with the filter 4 capped with the cap 6, the syringe 3 was driven to recover the lytic solution 5.

SDS removal

500μ l of the obtained eluate was transferred into a 1.5-ml microtest tube, and an equivalent amount of a 500 mM aqueous solution of KCl was added. The precipitating fraction was removed by centrifugation to yield an eluate.

Heat treatment

The obtained eluate was incubated at 95°C for 10 minutes to inactivate proteinase K.

(3) DNA amplification process

5μ l of the eluate described above was subjected to polymerase chain reaction [PCR, Saiki, R. K. et al., Science, 239, 487-491 (1988)], a method of specifically amplify the desired DNA, for 4 hours. The PCR was carried out under the following conditions.

100μ l of mineral oil, as an anti-evaporation agent, was layered on a 50 μ l mixture of 26.5 μ l of distilled water, 8μ l of dNTP (1.25 mM of each dNTP), 2.5μ l (20μ M) of each of two kinds of primer, 5 μ l of 10× buffer (Perkin-Elmer Cetus) and 2.5 units of Taq polymerase (Perkin-Elmer Cetus), and this layered product was set onto the DNA Thermal Cycler (Perkin-Elmer Cetus). The primers used were the publicly known oligonucleotides for Escherichia coli and Proteus vulgaris. The 10× buffer is a 100 mM Tris-HCl buffer (pH 9.0) containing 500 mM KCl and 15 mM $MgCl_2$.

Each temperature cycle for the PCR lasted for 5.7 minutes, comprising 1 minute of denaturation at 94°C, 1 minute of annealing at 37°C and 1 minute of chain elongation at 60°C, and this cycle was repeated 42 times.

(4) Detection process

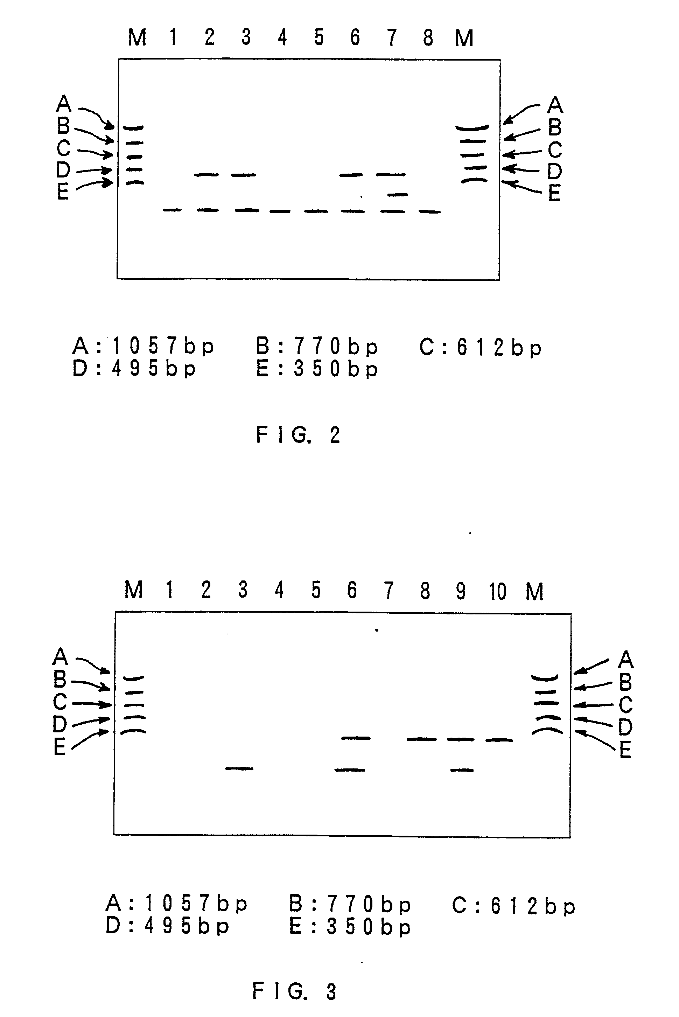

After 20 μ l of the PCR-treated solution described above was subjected to electrophoresis at 100 V on a 2% agarose gel containing ethidium bromide for 35 minutes, the gel was transferred onto a transilluminator and irradiated with UV light having a wavelength of 320 nm. As a result, the PCR-amplified DNA emitted fluorescence. This fluorescence was visualized using a camera loaded with an instant film. The results are shown in Figure 2. Lanes 1 and 5 show the patterns obtained by treating urine free of bacteria in the same manner as with the sample suspension. Lanes 2 and 6 show the patterns obtained by performing the present invention on Escherichia coli and Proteus vulgaris, respectively. Lanes 3 and 7 show the patterns obtained from the positive controls of respective bacteria. Lanes 4 and 8 show the patterns obtained by PCR in the absence of the eluate. M represents the molecular marker obtained by complete digestion of φ X174 DNA with the restriction enzyme Hinc; A, B, C and D represent the number of corresponding base pairs.

The electrophoretic pattern obtained after the method of the present invention is performed on a suspension of a sample (urine) containing Escherichia coli is shown in Lane 2 of Figure 2. As a result, a band appeared at 491 bp. Because this position is the same as the position of the positive control sample electrophoresed at the same time (Lane 3 of Figure 2), it is evident that Escherichia coli was successfully specifically detected. The positive control mentioned here is a DNA solution obtained by pure cultivating, for example, Escherichia coli ATCC 23519 in liquid medium, lysing it with enzyme or surfactant and then subjecting the lysed cells to phenol or chloroform extraction and ethanol precipitation. The same applies to the case of Proteus vulgaris.

The finding that a band of the same length (461 bp) as with the band from the positive control appeared demonstrates that the target bacterium was successfully specifically detected.

From the results shown above, it is evident that the method of bacterial examination of the present invention makes it possible to detect Escherichia coli or Proteus vulgaris conveniently and quickly in a short time within 5 hours on condition that at least $10^5$ cells of Escherichia coli or Proteus vulgaris are contained in each milliliter of urine.

Example 2

(1) Bacterial separation process

Preparation of sample suspension

0.4 g of human feces and $10^6$, $10^7$ or $10^8$/gram feces of Bacillus cereus JCM 2152 strain were suspended in 4 ml of a 50 mM phosphate buffer (pH 7.0) to yield a sample suspension.

Rough filtration

The sample suspension thus obtained was subjected to filtration. The sample suspension was placed in a 10-ml syringe and filtered through a nylon mesh having a pore size of 10 $\mu$ m attached to the pp-47 filter holder (Advantech) connected to the syringe.

(2) Bacterial cell component collection process

Lytic treatment

To the filtrate obtained above, N-acetylmuramidase and acromopeptidase were added to final concentrations of 60$\mu$ g/ml and 1 mg/ml, respectively, followed by incubation at 37 °C for 10 minutes. SDS and proteinase K were added to final concentrations of 1% and 1 mg/ml, respectively, followed by incubation at 60 °C for 30 minutes. Further inactivation was carried out at 95 °C for 10 minutes to inactivate the proteinase K.

Filtration

To the obtained solution, a 1/10 amount of an aqueous solution of 2.5 M KCl was added. After SDS component was coagulated, 2 ml of the solution was passed through the MILLEX-PF 0.8 $\mu$ m filter unit and MILLEX GV 0.22$\mu$ m filter unit to remove the insoluble fraction and SDS component.

Ethanol precipitation

To the obtained filtrate, a 1/10 amount of 3 M sodium acetate (pH 5.2) and a 2-fold amount of 99% cold ethanol were added.

Capturing the nucleic acid component

GF/D (glass fiber filter paper, produced by Whatman) was attached to a filter holder 4 mm in diameter (made by the inventor), through which the obtained solution was filtered. This filter was then washed with 70% cold ethanol and 99% cold ethanol.

Elution of nucleic acid component

The filter described above was irrigated with 25 $\mu$ l of an aqueous solution of 0.5% NONIDET P-40 and 0.5% Tween 20 using a 1-ml syringe. After incubation at 50 °C for 10 minutes, the syringe was driven to recover the nucleic acid component.

(3) DNA amplification process

5$\mu$ l of the eluate described above or a 10-fold or 100-fold dilution thereof was subjected to polymerase chain reaction for 4 hours. The PCR was carried out under the following conditions. 100$\mu$ l of mineral oil, as an anti-evaporation agent, was layered on a 50$\mu$ l mixture of 26.5 $\mu$ l of distilled water, 8 $\mu$ l of dNTP (1.25 mM of each dNTP), 2.5 $\mu$ l (20 $\mu$ m) of each of two kinds of primer, 5$\mu$ l of 10 × buffer (Perkin-Elmer Cetus) and 2.5 units of Taq polymerase (Perkin-Elmer Cetus), and this layered product was set onto the DNA Thermal Cycler (Perkin-Elmer Cetus). The 10 × buffer is a 100 mM Tris-HCl buffer (pH 9.0) containing 500 mM KCl and 15 mM MgCl$_2$.

The primers used were the following DNA base sequences ((a)~(e)) specific to Cereus bacteria;

(a)  (5')d-GGTTTAAGTATTACAAGCC(3')  (SEQ ID NO:1)

(b)  (5')d-GCATATACACCTAATCGAGC(3')  (SEQ ID NO:2)

(c)  (5')d-CCACTAAGTCTTCTTTCG(3')  (SEQ ID NO:3)

(d)  (5')d-TTCTGTATGCCCTTTCCCTG(3')  (SEQ ID NO:4)

(e)  (5')d-ATTTCAGAAGCGCGTAACGG(3')  (SEQ ID NO:5)

Every primer was synthesized using the automatic DNA synthesizer NS-1 (Shimadzu corporation) and purified using a high performance liquid chromatograph loaded with a reverse phase column.

Each temperature cycle for the PCR lasted for 5.7 minutes, comprising 1 minute of denaturation at 94°C, 1 minute of annealing at 37°C and 1 minute of chain elongation at 60°C, and this cycle was repeated 42 times.

(4) Detection process

After 20 $\mu$ l of the PCR-treated solution described above was subjected to electrophoresis at 100 V on a 2% agarose gel containing ethidium bromide for 35 minutes, the gel was transferred onto a transilluminator and irradiated with UV light having a wavelength of 320 nm. As a result, the PCR-amplified DNA emitted fluorescence. This fluorescence was visualized using a camera loaded with an instant film. The results are shown in Figure 3. The electrophoretic patterns obtained after performing the method of the present invention on suspensions of samples (feces) containing Bacillus cereus are shown in Lanes 1 through 9 of Figure 3. Lanes 1, 2 and 3 show the patterns obtained with fecal suspensions containing $10^6$ cells per gram feces; Lanes 4, 5 and 6 show the patterns obtained with fecal suspensions containing $10^7$ cells per gram feces; Lanes 7, 8 and 9 show the patterns obtained with fecal suspensions containing $10^8$ cells per gram feces. The patterns shown in Lanes 1, 4 and 7 were obtained by subjecting the original eluate to PCR; the patterns shown in Lanes 2, 5 and 8 were obtained by subjecting 10-fold dilutions of the original eluate to PCR; the patterns shown in Lanes 3, 6 and 9 were obtained by subjecting 100-fold dilutions of the original eluate to PCR. Lane 10 shows the pattern obtained with the positive control of Bacillus cereus. M represents the molecular marker obtained by complete digestion of $\phi$ x174 DNA with the restriction enzyme Hinc; A, B, C and D represent the number of corresponding base pairs.

As a result, a band appeared at about 232 bp. Because this position is the same as the position of the positive control sample electrophoresed at the same time (Lane 10 of Figure 3), it is evident that Bacillus cereus was successfully specifically detected. The positive control mentioned here is a DNA solution obtained by pure cultivating a standard strain of Bacillus cereus (JCM 2152 strain) in liquid medium, lysing it with enzyme or surfactant and then subjecting the lysed cells to phenol or chloroform extraction and ethanol precipitation.

The results shown above demonstrate that the method of bacterial examination of the present invention makes it possible to detect Bacillus cereus conveniently and quickly in a short time within 5 hours on condition that at least $10^6$ cells of Bacillus cereus are contained in each gram of feces.

SEQUENCE LISTING

SEQ ID NO:1

SEQUENCE LENGTH: 19 bases

SEQUENCE TYPE: nucleic acid

STRANDEDNESS: single

TOPOLOGY: linear

MOLECULE TYPE: genomic DNA

HYPOTHETICAL: No

ANTI-SENSE: No

ORIGINAL SOURCE:

    ORGANISM: Bacillus cereus

FEATURE:

    IDENTIFICATION METHOD: S

SEQUENCE

GGTTTAAGTA TTACAAGCC                    19

SEQ ID NO:2

SEQUENCE LENGTH: 20 bases

SEQUENCE TYPE: nucleic acid

STRANDEDNESS: single

TOPOLOGY: linear

MOLECULE TYPE: genomic DNA

HYPOTHETICAL: No

ANTI-SENSE: No

ORIGINAL SOURCE:

    ORGANISM: Bacillus cereus

FEATURE:

    IDENTIFICATION METHOD: S

SEQUENCE

GCATATACAC CTAATCGAGC                                    20


SEQ ID NO:3

SEQUENCE LENGTH: 18 bases

SEQUENCE TYPE: nucleic acid

STRANDEDNESS: single

TOPOLOGY: linear

MOLECULE TYPE: genomic DNA

HYPOTHETICAL: No

ANTI-SENSE: No

ORIGINAL SOURCE:

    ORGANISM: Bacillus cereus

FEATURE:

    IDENTIFICATION METHOD: S

SEQUENCE

CCACTAAGTC TTCTTTCG                                      18


SEQ ID NO:4

SEQUENCE LENGTH: 20 bases

SEQUENCE TYPE: nucleic acid

STRANDEDNESS: single

TOPOLOGY: linear

MOLECULE TYPE: genomic DNA

HYPOTHETICAL: No

ANTI-SENSE: No

ORIGINAL SOURCE:

    ORGANISM: Bacillus cereus

FEATURE:

    IDENTIFICATION METHOD: S

SEQUENCE

TTCTGTATGC CCTTTCCCTG                20


SEQ ID NO:5

SEQUENCE LENGTH: 20 bases

SEQUENCE TYPE: nucleic acid

STRANDEDNESS: single

TOPOLOGY: linear

MOLECULE TYPE: genomic DNA

HYPOTHETICAL: No

ANTI-SENSE: No

ORIGINAL SOURCE:

    ORGANISM: Bacillus cereus

FEATURE:

    IDENTIFICATION METHOD: S

SEQUENCE

ATTTCAGAAG CGCGTAACGG                20


**Claims**

1. A method of bacterial examination to determine whether or not a target bacterium is present in a sample which comprises collecting a bacterial cell component from a suspension containing bacteria in a filter, amplifying DNA contained in said bacterial cell component by polymerase chain reaction and detecting said amplified DNA.

2. The method according to claim 1, and further comprising a pretreatment for separation of insoluble solids in a sample.

3. The method according to claims 1 or 2 wherein said filter is composed of 2 kinds of glass fiber filter for trapping the bacteria in a sample.

4. The method according to any one of claims 1 to 3, wherein said target bacterium is a causative microorganism of urinary tract infection.

5. The method according to any one of claims 1 to 3, wherein said target bacterium is a causative microorganism of food poisoning.

6. An apparatus for bacterial examination which comprises means for collecting a bacterial cell component from a suspension containing bacteria in a filter, means for amplifying a DNA contained in said bacterial cell component by polymerase chain reaction and means for detecting said amplified DNA.

7. An apparatus for bacterial examination which comprises means for aspirating a sample solution into a syringe equipped with a filter, means for conveying said syringe on the treating line to collect a bacterial cell component from said sample solution, means for amplifying DNA contained in said bacterial cell component by polymerase chain reaction and means for detecting said amplified DNA.

8. The apparatus according to claims 6 or 7 wherein said means for amplifying the DNA comprises a group of vessels and three incubators controlled at a temperature suitable for polymerase chain reaction.

9. The apparatus according to any one of claims 6 to 8 wherein said means for detecting the amplified DNA is an electrophoretic apparatus.

FIG. 1

A : 1 0 5 7 b p     B : 7 7 0 b p     C : 6 1 2 b p
D : 4 9 5 b p       E : 3 5 0 b p

FIG. 2

A : 1 0 5 7 b p     B : 7 7 0 b p     C : 6 1 2 b p
D : 4 9 5 b p       E : 3 5 0 b p

FIG. 3

FIG. 4

EP 0 461 477 A1

F I G. 5

16    5min/step    18    18    18    18    2.5min/step    18

18 (37℃)    18 (60℃)    18 (95℃)    18 (0 ℃)    18 (60℃)

17    16    15    11    12    13    14    19    20

Collection & Washing process    Lytic treatment process    Precipitation process    Elution process    Output

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | "PCR Protocols: A guide to methods and applications", edited by M.A. Innis et al., 1989, pages 399-406, Academic Press, Inc., San Diego, US; R.M. ATLAS et al.: "Detecting bacterial pathogens in environmental water samples by using PCR and gene probes" <br> * The whole document, esp. page 402, line 2 - page 403, line 28 * | 1 | C 12 Q 1/68 |
| Y | JOURNAL OF CLINICAL MICROBIOLOGY, vol. 27, no. 2, February 1989, pages 261-265, American Society for Microbiology; D.M. OLIVE: "Detection of enterotoxigenic Escherichia coli after polymerase chain reaction amplification with a thermostable DNA polymerase" <br> * Abstract; page 261, column 2, line 33 - page 262, column 1, line 42; page 264, column 1, lines 57-65 * | 1 | |
| Y | WO-A-8 904 374 (OLIN CORP.) <br> * Page 2, line 20 - page 3, line 16; page 13, lines 23-31 * | 1 | |
| A | EP-A-0 114 668 (INTEGRATED GENETICS INC.) <br> * Page 4, line 14 - page 5, line 12; claim 12 * | 1,2 | TECHNICAL FIELDS SEARCHED (Int. Cl.5) |
| A | JOURNAL OF CLINICAL MICROBIOLOGY, vol. 27, no. 6, June 1989, pages 1257-1261, American Society for Microbiology; M.N. STARNBACH et al.: "Species-specific detection of Legionella pneumophila in water by DNA amplification and hybridization" <br> * Abstract; page 1258, column 1, lines 26-43; page 1260, column 1, line 35 - column 2, line 17 * | 1 | C 12 Q <br> G 01 N |
| P,X,L | WORLD PATENT ABSTRACTS, JAPANESE PATENTS ABSTRACTS, week 9029, 29th August 1990, A-M Chemical, page 12, abstract no. 90-221171/29, Derwent Publications Ltd, London, GB; <br> & JP-A-2 150 295 (SHIMADZU SEISAKUSHO K.K.) <br> * The whole abstract (casts doubts on priority) * | 1,6,7 | |

The present search report has been drawn up for all claims

| Place of search | Date of completion of search | Examiner |
|---|---|---|
| The Hague | 23 September 91 | LUZZATTO E.R.P.G.A. |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

&: member of the same patent family, corresponding document